# EUROPÄISCHE PATENTSCHRIFT

(11) **EP 2 983 621 B1**
(45) Veröffentlichungstag und Bekanntmachung des Hinweises auf die Patenterteilung: **20.12.2017**
(21) Anmeldenummer: 14715903.2
(22) Anmeldetag: 07.04.2014
(51) Int. Cl.: A61F 2/30, A61F 2/36, A61F 2/46

(54) **BEFESTIGUNG KERAMISCHER BAUTEILE**
FASTENING CERAMIC COMPONENTS
FIXATION DE COMPOSANTS EN CÉRAMIQUE

(30) Priorität: 08.04.2013 DE 102013005862; 27.06.2013 DE 102013212456
(43) Veröffentlichungstag der Anmeldung: 17.02.2016
(73) Patentinhaber: CeramTec GmbH, 73207 Plochingen (DE)
(72) Erfinder: WECKER, Heinrich, 90542 Eckental (DE); KELNBERGER, Alfons, 90552 Röthenbach (DE); KRAUSE, Christoph, 73760 Ostfildern (DE); ZIERMANN, Frank, 10967 Berlin (DE)
(74) Vertreter: Uppena, Franz
(86) Internationale Anmeldenummer: PCT/EP2014/056947
(87) Internationale Veröffentlichungsnummer: WO 2014/166877

(56) Entgegenhaltungen:
- WO-A2-2012/125795
- FR-A1- 2 261 743
- FR-A1- 2 391 711
- FR-A1- 2 832 625
- FR-A2- 2 310 120
- US-A- 4 995 883

## Beschreibung

Die Erfindung betrifft die Befestigung von keramischen Bauteilen an anderen Bauteilen. Insbesondere betrifft die Erfindung die Befestigung von keramischen Prothesen-Bauteilen, an anderen, insbesondere metallischen Prothesen-Teilen.

Aufgrund ihrer besonderen Eigenschaften können Bauteile aus keramischen Materialien nicht wie beispielsweise metallische Bauteile einfach mit anderen Bauteilen verschraubt oder in ähnlicher Weise mit anderen Bauteilen verbunden werden. Keramische Bauteile sind insbesondere bei punktuellen Belastungen sehr bruchanfällig. Beispielsweise wirken bei verschraubten Verbindungen große Kräfte auf die kleinen Kontaktflächen der Anpressfläche der Schraube an dem keramischen Bauteil. Schon diese Belastung kann zu hohen lokalen Spannungen an diesen Kontaktbereichen und in der Folge zum Versagen der Keramik durch Bruch führen. Darüber hinaus besteht eine Kerbwirkung an scharfen keramischen Bauteil-Kanten, wie sie beispielsweise bei versenkten Schrauben notwendig entstehen. Diese Kanten können daher Ausgangspunkte von Rissen sein, die jedenfalls mittelfristig zum Versagen des Bauteils führen können.

Insbesondere wenn es sich bei den keramischen Bauteilen, die mit anderen Bauteilen verbunden werden sollen, um Teile von Prothesen und insbesondere um Teile von Endoprothesen handelt, kann ein solches Versagen des keramischen Bauteils nicht toleriert werden.

Diese Problematik ist als solche bekannt. Im Prothesen-Bau werden häufig metallische Teile, insbesondere aus Titan und Titanlegierungen, verwendet, die problemlos miteinander verschraubt werden können, um eine sichere Verbindung zwischen verschiedenen Bauteilen zu gewährleisten.

Keramische Bauteile werden oftmals mit anderen Bauteilen verklemmt. Ein Beispiel für eine solche Klemmung ist die konische Klemmung, die beispielsweise aus der DE 43 35 931 B4 bekannt ist. Hier wird ein keramisches Bauteil mit einer konischen Außenwandung in ein metallisches Bauteil mit einer konischen Öffnung geklemmt, wobei der Konus des keramischen Bauteils einen anderen Winkel als der Konus des metallischen Bauteils aufweisen kann.
Die konische Klemmung ermöglicht einen relativ großen Kontaktbereich durch den punktuelle Belastungen vermieden werden können. Allerdings hat sie den Nachteil, dass die Verbindung der Bauteile nicht gegen eine spätere Lockerung gesichert werden kann. Dies kann insbesondere dann problematisch sein, wenn das keramische Bauteil Teil einer modularen Endoprothese ist, die erst bei Implantieren im Körper des Patienten zusammengesetzt werden kann und deren Verbindungsfestigkeit vom Operateur abhängig ist. Solche modularen Prothesen finden beispielsweise als Hüftgelenks-, Schultergelenksprothesen oder auch anderen kleinen Gelenksprothesen Verwendung. Die modulare Bauform umfasst dann einen beispielsweise metallischen Schaft, der in den Oberschenkel- oder den Oberarmknochen eingeschlagen wird. Auf diesen Schaft wird eine keramische Kugel als Ersatz für den Oberschenkel- bzw. Oberarmkopf aufgesetzt. Die Verbindung beider Komponenten erfolgt durch Aufschlagen oder Impaktierung des auf den Schaft aufgesetzten Kopfes. Um eine sichere, kraftschlüssige Verbindung zu erreichen, muss die Impaktierung jedoch mit einer genau dosierten Kraft und unter genauer Passung erfolgen. Ist die Passung nicht optimal oder die Impaktierkraft zu niedrig, kann sich der keramische Kopf lockern. Eine gesicherte feste Verbindung, beispielsweise eine Schraubverbindung, zwischen den beiden Prothesen-Teilen wäre daher wünschenswert.

Der nächstliegende Stand der Technik wird in FR2391711 offenbart. Die Aufgabe der Erfindung besteht darin, eine gesicherte, feste Verbindung eines keramischen Bauteils mit einem weiteren Bauteil zur Verfügung zu stellen.
Die Aufgabe der Erfindung wird durch einen Gegenstand mit den Merkmalen des Anspruchs 1 gelöst. Danach umfasst eine feste Verbindung eines keramischen Bauteils mit einem weiteren Bauteil das keramische Bauteil mit einem ersten Verbindungselement, über das die Verbindung zu dem weiteren Bauteil hergestellt werden kann. Es handelt sich bei der Verbindung zwischen dem keramischen Bauteil und dem weiteren Bauteil um eine kraftschlüssige Verbindung, insbesondere um eine Schraubverbindung.
Unter einer festen Verbindung im Sinne der Erfindung wird eine Verbindung verstanden, die das keramische und das weitere Bauteil zu einer Einheit zusammenfügt, so dass die Einheit bei bestimmungsgemäßer Verwendung auch verbunden bleibt.
Anders ausgedrückt betrifft die Erfindung also eine Bauteilkombination aus einem keramischen Bauteil und einem weiteren Bauteil, die fest miteinander verbunden sind, wobei das keramische Bauteil mit einem ersten Verbindungselement verbunden ist, und das weitere Bauteil ein zweites Verbindungselement aufweist. Die Verbindung zwischen dem keramischen und dem anderen Bauteil erfolgt dabei ausschließlich durch die Verbindung des ersten mit dem zweiten Verbindungselement.
Eine solche Konstruktion ist immer dann vorteilhaft, wenn beispielsweise das Verbinden zweier Bauteile durch eine konische Klemmung nicht unter optimalen Bedingungen ausgeführt werden kann oder, wenn eine lösbare Verbindung zwischen den beiden Bauteilen gewünscht ist. Dies ist insbesondere der Fall, wenn es sich bei den Bauteilen um Teile einer modularen Prothese, insbesondere einer Knie-, Hüft- oder Schultergelenks-Prothese handelt. Unter einer modularen Prothese wird im Rahmen dieser Erfindung eine Prothese verstanden, die aus verschiedenen Bauteilen besteht, die besonders bevorzugt erst beim Implantieren durch den behandelnden Arzt zusammengesetzt werden. Das Zusammensetzen kann sowohl im Körper des Patienten als auch direkt vor der Implantation erfolgen. Dies hat den Vorteil, dass sperrige Prothesen in kleineren Einzelteilen implantiert werden können und, dass die Bauteile der Anatomie des Patienten entsprechend zusammengestellt werden können.

Gemäß der Erfindung umfasst das erste Verbindungselement ein Metall oder eine Metall-Legierung, bevorzugt Titan oder Titan-Legierungen. Das erste Verbindungselement kann mit dem keramischen Bauteil bevorzugt kraftschlüssig verbunden sein. Diese kraftschlüssige Verbindung kann bevorzugt eine konische Klemmung sein. Gemäß der Erfindung umfasst das erste Verbindungselement eine Hülse. Unter einer Hülse wird im Rahmen dieser Erfindung ein zylinderförmiger oder konischer Körper verstanden, der hohl oder massiv sein kann. Jedenfalls eine Stirnfläche des Körpers ist geschlossen. Zumindest in dieser Stirnfläche ist eine Öffnung vorgesehen, so dass eine Schraube oder Ähnliches hindurchgesteckt werden und mit dem weiteren Bauteil verschraubt oder verbunden werden kann. Wenn der Körper massiv ist, weist er eine durchgehende Bohrung auf, die durch beide Stirnflächen des Körpers führt. Die Öffnung in der Stirnfläche oder die Bohrung können bevorzugt zentriert und/oder entlang der Rotationsachse des Körpers vorgesehen sein. Gemäß einer anderen Ausführungsform der Erfindung kann aber auch eine exzentrische Anordnung zweckmäßig sein, um eine winklige Anordnung zwischen den Bauteilen zu ermöglichen.
Gemäß noch einer Weiterbildung der Erfindung kann die Hülse ein Innengewinde umfassen, in das eine Schraube eingeschraubt werden kann.
Das weitere Bauteil kann gemäß einer bevorzugten Ausführungsform der Erfindung der Schaft einer Prothese sein. Bevorzugte Materialien für das weitere Bauteil umfassen Metalle und Metall-Legierungen, insbesondere Titan und Titan-Legierungen. Andere Materialien sind je nach Verwendung der Bauteilkombination natürlich ebenfalls möglich.
Gemäß einer besonders bevorzugten Ausführungsform der Erfindung kann eine Prothese für Hüft- und Schultergelenke beispielsweise einen keramischen Kopf mit einer Ausnehmung umfassen. In die Ausnehmung ist eine Hülse kraftschlüssig eingepasst. Die kraftschlüssige Passung kann bevorzugt durch eine konische Klemmung der Komponenten erreicht werden. Jede andere kraftschlüssige Verbindung, die die speziellen Probleme bei der Verbindung von keramischen Materialien mit anderen Materialien berücksichtigt, ist jedoch ebenfalls möglich. Gemäß einer besonders bevorzugten Ausführungsform der Erfindung kann die Hülse ein Innengewinde aufweisen. Ist die Verbindung zwischen dem keramischen Bauteil und dem weiteren Bauteil lösbar ausgestaltet, kann das Gewinde als Revisionsgewinde vorgesehen sein, also nur dann zum Einsatz kommen, wenn ein späterer Austausch des Kugelkopfes notwendig wird. Dann kann das auszutauschende keramische Bauteil, z.B. ein Prothesenkopf, mittels dieses Gewindes von dem weiteren Bauteil, z.B. dem Prothesenschaft, abgezogen werden. Diese Ausführungsform hat den Vorteil, dass beim Austausch des keramischen Bauteils praktisch keine mechanische Belastung auf die Verbindung des Prothesenschafts mit dem Knochen ausgeübt werden muss. Daher kommt es auch nicht zu einer Lockerung dieser Verbindung infolge einer Austauschoperation.

Das weitere Bauteil umfasst gemäß der Erfindung ein zweites Verbindungselement, das als Gegenstück zu dem ersten Verbindungselement des keramischen Bauteils fungiert. Ist eine Schraubverbindung vorgesehen, kann das zweite Verbindungselement beispielsweise ein Sackloch mit einem Innengewinde sein, wenn das zweite Verbindungselement integral mit dem weiteren Bauteil ausgeführt ist.
Besonders bevorzugt ist eine Ausführungsform, bei der das erste und das zweite Verbindungselement jeweils ein Gewinde aufweisen, jedoch mit unterschiedlichem Gewindegang und/oder Gewindedurchmesser. So kann, beispielsweise mittels passender Schrauben bei bestimmungsgemäßem Gebrauch eine Sicherung der Bauteile gegen ein unerwünschtes Lösen erreicht werden. Sollte aber doch eine Revisionsoperation notwendig werden, kann das Gewinde des ersten Verbindungselements trotzdem als Revisionsgewinde verwendet werden.

Diese Ausführungsform hat den Vorteil, dass die Schraube definiert geführt und winkelstabil ist. Eine Auflagefläche ist nicht notwendig, die Anpresskraft ist höher.
Das zweite Verbindungselement kann jedoch auch ein separates Bauteil sein, das fest mit dem weiteren Bauteil verbunden ist. Das zweite Verbindungselement kann beispielsweise ein Zapfen mit oder ohne eine Basisplatte sein, wobei der Zapfen formschlüssig in die Hülse bzw. das erste Verbindungselement passt.
Die Verbindung zwischen erstem und zweitem Verbindungselement kann dann kraftschlüssig, beispielsweise durch eine konische Klemmung des Zapfens in der Hülse hergestellt werden. Alternativ kann der Zapfen auch ein Innengewinde aufweisen, so dass die beiden Bauteile durch Verschrauben verbindbar sind. Die Erfindung sieht vor, dass das keramische Bauteil (1) eine durchgehende Öffnung aufweist, die mit einer Öffnung in der Hülse (2) fluchtet. Die Öffnung der Hülse weist dabei einen geringeren Durchmesser als die Öffnung des keramischen Bauteils auf, so dass ein vorstehender Rand der Hülse als Widerlager für ein Befestigungselement, beispielsweise eine Schraube, dienen kann.
Ist das weitere Bauteil der Schaft einer modularen Prothese, so kann dieser ein Sackloch mit einem Innengewinde aufweisen. Auf diese Weise kann der Schaft der Prothese ohne besondere Rücksicht auf das Material in den Knochen eingeschlagen werden. Der keramische Kugelkopf der Prothese wird anschließend auf den Schaft aufgesetzt und mittels einer Schraube, die durch ein Loch im keramischen Kopf in die Hülse eingesetzt wird, mit dem Schaft verschraubt.
Dies hat den Vorteil, dass die Hülse als Führung beim Aufsetzen des Kopfes auf den Schaft und gleichzeitig als Widerlager für die Schraube dienen kann. Es besteht also kein direkter Kontakt des keramischen Bauteils mit der Schraube, so dass eine Punktbelastung des keramischen Bauteils durch die Schraube ausgeschlossen werden kann. Darüber hinaus kann der keramische Kopf mit einer definierten Kraft und in einem definierten Winkel mit dem Schaft verbunden werden. Dies steht im Gegensatz zu der gängigen Verbindung von Keramikkopf und Schaft mittels konischer Klemmung, die durch Impaktierung hergestellt wird.

Ein weiterer Vorteil besteht darin, dass der keramische Kopf im Falle eines Bauteilversagens durch einfaches Abschrauben vom Schaft ausgetauscht werden kann. Im Gegensatz zu den derzeit gängigen Verfahren, bei denen der kaputte Kopf vom Schaft abgeschlagen werden muss, ist mit der hier vorgestellten Prothese ein Austausch ohne mechanische Belastung für die Verankerung des Schafts im Knochen möglich. Dies verhindert vorteilhaft die Lockerung des eingewachsenen Schafts im Knochen.

Ein weiterer Vorteil einer solchen Verbindung eines keramischen Bauteils mit einem weiteren Bauteil ist die gesicherte Verbindung zwischen den Bauteilen. Eine solche gesicherte Verbindung ist durch eine konische Klemmung nicht immer zu erreichen. Die konische Klemmung, die durch den Operateur durch Impaktieren des Kopfes auf den Schaft bei der Implantation vorgenommen wird, weist notwendig einige Unsicherheitsfaktoren auf. Zum einen muss der keramische Kopf der Prothese im richtigen Winkel aufgeschlagen werden, weil es ansonsten zu einer ungleichmäßigen Belastung der Klemmfläche und damit zu den gefürchteten Punktbelastungen der Keramik kommen kann. Darüber hinaus hat eine ungenaue Passung natürlich auch Auswirkungen auf die Funktion der Prothese. Außerdem muss beim Impaktieren ein Impuls einer bestimmten Stärke auf den Kopf wirken, damit es zu einer vollständigen Klemmung kommt. Beide Faktoren, also Winkel und Impuls, hängen vom Können des Operateurs ab und sind damit fehleranfällig.

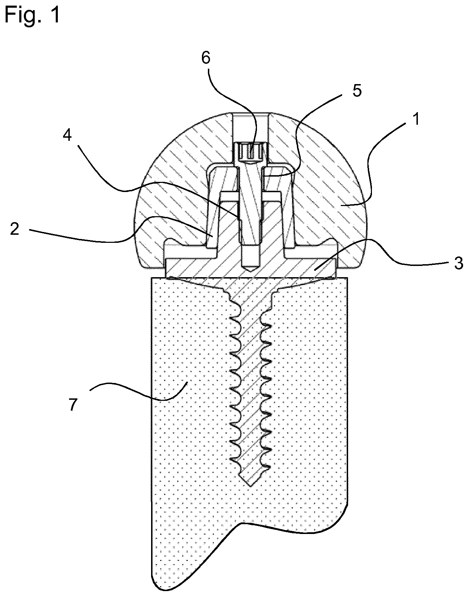

Im Folgenden wird die Erfindung anhand einer Ausführungsform der Erfindung näher erläutert. Figur 1 zeigt einen Querschnitt durch eine Hüft- oder Schultergelenksprothese, die einen modularen Aufbau aufweist. Auf einen metallischen Schaft 7 ist eine keramische Kugel 1 montiert.

Die keramische Kugel 1 umfasst eine metallische Hülse 2, wobei die Hülse bevorzugt aus Titan besteht. Die metallische Hülse 2 ist werksseitig mit der keramischen Kugel 1 kraftschlüssig, beispielsweise durch eine konische Klemmung, verbunden. Die keramische Kugel 1 weist eine durchgängige Öffnung auf, die mit der Öffnung in der Stirnfläche der Hülse 2 fluchtet. Die Öffnung in der Stirnfläche der Hülse 2 hat jedoch einen geringeren Durchmesser als die Öffnung in der keramischen Kugel 1, so dass vorstehende Oberseite der Hülse als Widerlager für einen Schraubenkopf dienen kann. Die Öffnung in der keramischen Kugel 1 kann zentrisch oder auch exzentrisch angeordnet sein.

Auf den Schaft 2 ist eine metallische Basisplatte 3 montiert, die einen zylindrischen Vorsprung oder Zapfen mit einer Gewindebohrung 4 aufweist. Die Dimensionen der Hülse 2 und des zylindrischen Vorsprungs der Basisplatte sind aufeinander abgestimmt, so dass die keramische Kugel 1 mittels der Hülse 2 formschlüssig auf den Vorsprung aufgesetzt werden kann.

Die Verbindung zwischen keramischer Kugel 1 und Schaft 7 ist durch eine Schraube 6 gesichert, die durch die Öffnung der keramischen Kugel 1 und durch die Hülse 2 in das Innengewinde des zylindrischen Vorsprungs der Basisplatte 3 geschraubt ist. Die vorstehende Oberseite der Hülse 2 dient als Widerlager für den Schraubenkopf. So erfolgt die kraftschlüssige Verbindung nicht direkt über das keramische Material, sondern mit der Oberfläche der metallischen Hülse 2. Damit werden unkontrollierte punktförmige Belastungen des keramischen Materials vermieden.

Gemäß einer besonderen Ausführungsform der Erfindung kann die Hülse 2 ebenfalls ein Innengewinde 5 aufweisen, das als Revisionsgewinde fungieren kann. Das Innengewinde 5 weist vorteilhaft einen größeren Durchmesser auf als das Innengewinde, mit dem das keramische Bauteil regelrecht mit dem anderen Bauteil verbunden ist. Dann kann im Revisionsfall der keramische Kopf durch Einschrauben einer größeren Schraube vom Schaft einfach abgezogen werden, ohne, dass eine mechanische Belastung auf die Verwachsung des Schafts mit dem Knochen ausgeübt wird.

Im Folgenden werden nochmals die Vorteile der vorliegenden Erfindung aufgelistet:
- Sichere Verbindungen sind aus dem Bereich der Hüfte bekannt, jedoch sind die anwendbaren Kräfte bei der Impaktierung insbesondere nicht auf Schulterimplantate übertragbar. Die konische Verbindung zwischen keramischer Schulterkomponente und Metallschaft könnte theoretisch ausreichen, evtl. wird aber aus Unsicherheit des Arztes oder den schwächeren anatomischen Strukturen geschuldet, nicht fest genug impaktiert.
- In rein metallischen Systemen wurde diese Unsicherheit durch eine zusätzliche Sicherungsschraube zwischen Kugelkopf und Sockel gedeckelt - das ist an der Schnittstelle zwischen Metall und Keramik nicht möglich
- Impaktierung und modularer Aufbau einer Schulter-Prothese ist aus OPtechnischen Gründen zwingend notwendig (Ausrichtung der Winkel und des Offsets)
- Weiterer Nachteil: Knochenzement, Blut, Fett, Knochenpartikel etc. im Winkelspalt der konischen Klemmung reduzieren die Bauteilfestigkeit der keramischen Komponenten deutlich.
- Lösungsvorschlag: Eine werksseitig vorgepresste Titanhülse 2 wird fest mit dem Keramikkonus 1 verbunden. Diese Titanhülse 2 hat eine Oberflächenstruktur, die aus der Hüftbereich bekannt und bewährt ist. Zusätzlich enthält sie ein Gewinde 5 für den Revisionsfall im oberen Bereich. Eine Fixierschraube 6 presst und zentriert dabei die Keramikkomponente 1 mit der Titanhülse 2 auf dem Konus der Basisplatte 3 an einem Fixiergewinde 4.
- Vorteil 1: Wesentlich höhere, definierte Verbindungskräfte sind durch die werkseitige Montage Keramik-Hülse erreichbar
- Vorteil 2: Keine zusätzliche Sicherung der Verbindung Keramik-Metallhülse notwendig
- Vorteil 3: durch die werksseitige Vormontage wird die Fehlerquelle "intraoperative Verunreinigung" etc. ausgeschlossen
- Vorteil 4: weder Fretting (Abrieb) noch Korrosion zwischen der materialgleichen Hülse und Basisplatte möglich (im Vergleich zur rein metallischen Prothesenlösung)
- Vorteil 5: Über das Revisionsgewinde kann mit einem Revisionsinstrument der Kugelkopf mit der Titanhülse zerstörungsfrei und ohne Lockerung des Schaftes explantiert werden ohne dabei schlagende Krafteinwirkung auf die anatomischen Gegebenheiten anwenden zu müssen (vergleiche Abziehen einer Fahrradkurbel)
- Vorteil 6: Reduktion des Bruchrisikos durch die Fixierung des Systems Kugelkopf/Titanhülse auf der Basisplatte mit einer Schraube, die in das Fixiergewinde eingeschraubt wird, da ein direkter Kontakt des Schraubenkopfes mit der keramischen Komponente vermieden werden kann (keine Punktbelastungen, Bruchgefährdung an der Keramik)
- Vorteil 7: Wegen der schlechten intraoperativen Sichtverhältnisse und Zugänglichkeit (anatomische Lokalisation) dient die Schrauben-Hülsen-Kombination als Führungsmechanismus zum sicheren Platzieren auf der Basisplatte.
- Vorteil 8: weder Fretting noch Korrosion zwischen der Titanhülse und der Keramik möglich
- Vorteil 9: eine Hülsengröße ist mit allen Größen und Designvarianten der Keramikkugel kompatibel und kann universell verwendet werden (Reduktion des Fehlerrisikos während der Anwendung)
- Vorteil 10: Die Öffnung in der keramischen Komponente dient als Reservoir für Gelenksflüssigkeit (Synovia) zur Reduktion des Abriebes, insbesondere wenn Schalen aus PE als Gelenkpartner verwendet werden

## Patentansprüche

1. Feste Verbindung eines keramischen Bauteils (1) mit einem weiteren Bauteil (7), umfassend das keramische Bauteil (1), das mit einem ersten Verbindungselement (2) verbunden ist sowie das weitere Bauteil (7) mit einem zweiten Verbindungselement (3), wobei die Verbindung über ein Befestigungselement (6) erfolgt, wobei das erste Verbindungselement (2) eine Hülse umfasst und wobei das keramische Bauteil (1) eine durchgehende Öffnung aufweist, die mit einer Öffnung in einer Stirnfläche der Hülse (2) fluchtet, wobei die Öffnung in der Stirnfläche der Hülse (2) einen geringeren Durchmesser als die Öffnung des keramischen Bauteils (1) aufweist, so dass ein vorstehender Rand der Hülse (2) als Widerlager für ein Befestigungselement (6) dient, **dadurch gekennzeichnet, dass** das erste Verbindungselement (2) ein Metall oder eine Metall-Legierung umfasst, und dass die Verbindung über das Befestigungselement ausschließlich zwischen dem ersten (2) und dem zweiten Verbindungselement, unter Umgehung des keramischen Bauteils (1) erfolgt.

2. Feste Verbindung nach Anspruch 1, **dadurch gekennzeichnet, dass** das keramischen Bauteil (1) mit dem ersten Verbindungselement (2) kraftschlüssig, bevorzugt mittels einer konischen Klemmung, verbunden ist.

3. Feste Verbindung nach Anspruch 1 oder 2, **dadurch gekennzeichnet, dass** zweite Verbindungselement (3) integral mit dem weiteren Bauteil (7) ausgeführt ist.

4. Feste Verbindung nach Anspruch 1 oder 2, **dadurch gekennzeichnet, dass** das zweite Verbindungselement (3) fest mit dem weiteren Bauteil (7) verbunden ist.

5. Feste Verbindung nach Anspruch 1, **dadurch gekennzeichnet, dass** die Verbindung zwischen dem keramischen Bauteil (1) und dem weiteren Bauteil (7) lösbar ist.

6. Feste Verbindung nach dem vorstehenden Anspruch, **dadurch gekennzeichnet, dass** das zweite Verbindungselement (3) einen Zapfen umfasst, der formschlüssig in die Hülse (2) passt.

7. Feste Verbindung nach einem der vorstehenden Ansprüche, **dadurch gekennzeichnet, dass** das erste Verbindungselement (2) kraftschlüssig mit dem zweiten Verbindungselement (3) verbunden ist, bevorzugt mittels einer konischen Klemmung.

8. Feste Verbindung nach Anspruch 6 oder 7, **dadurch gekennzeichnet, dass** der Zapfen (3) ein Innengewinde (4) aufweist.

9. Feste Verbindung nach Anspruch 1, **dadurch gekennzeichnet, dass** das Befestigungselement (6) eine Schraube ist.

10. Feste Verbindung nach einem Ansprüche 1 bis 9, **dadurch gekennzeichnet, dass** die Hülse (2), insbesondere die Öffnung in der Stirnfläche der Hülse ein Innengewinde (5) aufweist.

11. Endoprothese, umfassend ein keramisches Bauteil (1) und ein weiteres Bauteil (7), **dadurch gekennzeichnet, dass** das keramische Bauteil (1) mit dem weiteren Bauteil (7) mittels einer festen Verbindung gemäß einem der Ansprüche 1 bis 10 verbunden ist.

## Claims

1. A firm connection of a ceramic component (1) to a further component (7), comprising the ceramic component (1), which is connected to a first connecting element (2), and also the further component (7) with a second connecting element (3), wherein the connection is effected by way of a fastening element (6), wherein the first connecting element (2) comprises a sleeve, and wherein the ceramic component (1) has a through-opening which is in alignment with an opening in an end face of the sleeve (2), wherein the opening in the end face of the sleeve (2) has a smaller diameter than the opening of the ceramic component (1) so that a projecting edge of the sleeve (2) serves as an abutment for a fastening element (6), **characterised in that** the first connecting element (2) comprises a metal or a metal alloy, and **in that** the connection by way of the fastening element is effected exclusively between the first connecting element (2) and the second connecting element, by-passing the ceramic component (1).

2. A firm connection according to claim 1, **characterised in that** the ceramic component (1) is connected to the first connecting element (2) in a force-locking manner, preferably by means of conical clamping.

3. A firm connection according to claim 1 or 2, **characterised in that** the second connecting element (3) is designed integrally with the further component (7).

4. A firm connection according to claim 1 or 2, **characterised in that** the second connecting element (3) is firmly connected to the further component (7).

5. A firm connection according to claim 1, **characterised in that** the connection between the ceramic component (1) and the further component (7) is releasable.

6. A firm connection according to the preceding claim, **characterised in that** the second connecting element (3) comprises a pin which fits into the sleeve (2) in a form-locking manner.

7. A firm connection according to one of the preceding claims, **characterised in that** the first connecting element (2) is connected to the second connecting element (3) in a force-locking manner, preferably by means of conical clamping.

8. A firm connection according to claim 6 or 7, **characterised in that** the pin (3) has an internal thread (4).

9. A firm connection according to claim 1, **characterised in that** the fastening element (6) is a screw.

10. A firm connection according to one of claims 1 to 9, **characterised in that** the sleeve (2), in particular the opening in the end face of the sleeve, has an internal thread (5).

11. An endoprosthesis, comprising a ceramic component (1) and a further component (7), **characterised in that** the ceramic component (1) is connected to the further component (7) by means of a firm connection in accordance with one of claims 1 to 10.

## Revendications

1. Liaison fixe d'un composant en céramique (1) avec un autre composant (7), comprenant le composant en céramique (1), qui est relié à un premier élément de liaison (2), ainsi que l'autre composant (7) avec un deuxième élément de liaison (3), sachant que la liaison est réalisée par l'intermédiaire d'un élément de fixation (6), sachant que le premier élément de liaison (2) comprend une douille et que le composant en céramique (1) présente une ouverture traversante qui est alignée avec une ouverture dans une face frontale de la douille (2), sachant que l'ouverture dans la face frontale de la douille (2) présente un diamètre plus faible que l'ouverture du composant en céramique (1), de sorte qu'un rebord saillant de la douille (2) sert de contre-appui à un élément de fixation (6),
**caractérisée en ce que** le premier élément de liaison (2) comporte un métal ou un alliage métallique et que la liaison par l'intermédiaire de l'élément de fixation se fait exclusivement entre le premier (2) et le deuxième élément de liaison, en contournant le composant en céramique (1).

2. Liaison fixe selon la revendication 1, **caractérisée en ce que** le composant en céramique (1) est relié au premier élément de liaison (2) par complémentarité de formes, de préférence au moyen d'un serrage conique.

3. Liaison fixe selon la revendication 1 ou 2, **caractérisée en ce que** le deuxième élément de liaison (3) est réalisé d'une seule pièce avec l'autre composant (7).

4. Liaison fixe selon la revendication 1 ou 2, **caractérisée en ce que** le deuxième élément de liaison (3) est relié de manière fixe à l'autre composant (7).

5. Liaison fixe selon la revendication 1, **caractérisée en ce que** la liaison entre le composant en céramique (1) et l'autre composant (7) est libérable.

6. Liaison fixe selon la revendication précédente, **caractérisée en ce que** le deuxième élément de liaison (3) comporte un tenon qui s'ajuste par complémentarité de formes dans la douille (2).

7. Liaison fixe selon l'une des revendications précédentes, **caractérisée en ce que** le premier élément de liaison (2) est relié par adhérence au deuxième élément de liaison (3), de préférence au moyen d'un emmanchement conique.

8. Liaison fixe selon la revendication 6 ou 7, **caractérisée en ce que** le tenon (3) présente un taraudage (4).

9. Liaison fixe selon la revendication 1, **caractérisée en ce que** l'élément de fixation (6) est une vis.

10. Liaison fixe selon l'une des revendications 1 à 9, **caractérisée en ce que** la douille (2), en particulier l'ouverture dans la face frontale de la douille, présente un taraudage (5).

11. Endoprothèse, comprenant un composant en céramique (1) et un autre composant (7), **caractérisée en ce que** le composant en céramique (1) est relié à l'autre composant (7) au moyen d'une liaison fixe selon l'une des revendications 1 à 10.
